# EUROPEAN PATENT SPECIFICATION

(11) **EP 4 147 661 B1**
(45) Date of publication and mention of the grant of the patent: **11.10.2023**
(21) Application number: 21195732.9
(22) Date of filing: 09.09.2021
(51) Int. Cl.: A61B 34/10, A61B 34/20, A61B 90/00, A61B 17/70, A61B 90/50, A61B 17/88, A61B 17/56

(54) **TECHNIQUE FOR COMPUTER-ASSISTED PLANNING OF PLACEMENT OF FASTENERS IN VERTEBRAE THAT ARE TO BE STABILIZED BY A PRE-FORMED SPINAL ROD**
TECHNIK ZUR RECHNERGESTÜTZTEN PLANUNG DER PLATZIERUNG VON BEFESTIGUNGSELEMENTEN IN DURCH EINEN VORGEFORMTEN WIRBELSÄULENSTAB ZU STABILISIERENDEN WIRBELN
TECHNIQUE DE PLANIFICATION ASSISTÉE PAR ORDINATEUR DE POSITIONNEMENT DE FIXATIONS DANS DES VERTÈBRES DEVANT ÊTRE STABILISÉES PAR UNE TIGE SPINALE PRÉFORMÉE

(43) Date of publication of application: 15.03.2023
(73) Proprietor: Stryker European Operations Limited, Carrigtwohill, Co. Cork T45 HX08 (IE)
(72) Inventor: HERRMANN, Florian, 77963 Schwanau (DE)
(74) Representative: Röthinger, Rainer

(56) References cited:
- WO-A1-2020/079598
- US-A1- 2019 336 179
- AGARWAL AAKASH ET AL: "Towards a validated patient-specific computational modeling framework to identify failure regions in traditional growing rods in patients with early onset scoliosis", NORTH AMERICAN SPINE SOCIETY JOURNAL (NASSJ), [Online] vol. 5, 1 March 2021 (2021-03-01), page 100043, XP055894389, ISSN: 2666-5484, DOI: 10.1016/j.xnsj.2020.100043 Retrieved from the Internet: URL:https://www.sciencedirect.com/science/ article/pii/S2666548420300433/pdfft?md5=7e a1326ed7df3005103ea7ee9f165ff1&pid=1-s2.0- S2666548420300433-main.pdf> [retrieved on 2022-02-22]

## Description

### Technical Field

The present disclosure generally relates to surgical planning. In more detail, a technique for computer-assisted planning of fastener placement in vertebrae that are to be stabilized by a pre-formed spinal rod is provided, wherein two or more fasteners are to couple the rod to the vertebrae. The technique may be implemented as a method, a computer-program product or an apparatus

### Background

Spinal interventions have become a widespread surgical treatment and are currently performed either manually by a surgeon, automatically by a surgical robot, or semiautomatically by a surgeon using robotic assistance. To guarantee proper surgical results, spinal interventions require surgical planning.

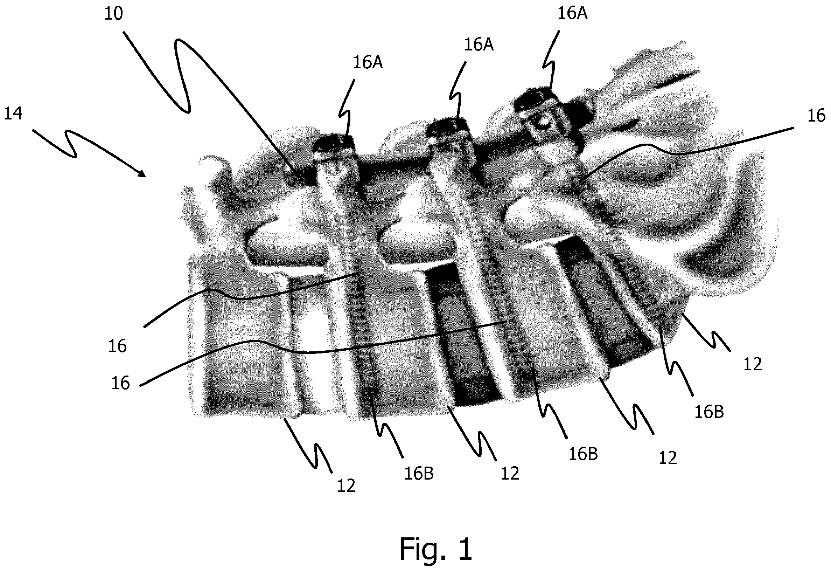

As illustrated in Fig. 1, in some spinal interventions a pre-formed rod 10 is implanted to stabilize two or more vertebrae 12 of a patient's spine 14. The rod 10 is preformed prior to its implantation according to a planned shape of the spine 14. After implantation, the rod 10 stabilizes the spine in accordance with the planned shape.

Fig. 1 shows that the pre-formed rod 10 is coupled to the vertebrae 12 using fasteners 16 such as bone screws (e.g., pedicle screws) having first ends 16A attached to the rod and opposite second ends 16B anchored in the vertebrae 12 along dedicated fastener extensions. The fastener extensions may have been planned by the surgeon prior to the spinal intervention based on pre-operatively acquired image data of the spine 14, such as computer tomography (CT) data. During the spinal intervention, computer-assisted navigation techniques may be used to guide fastener placement along trajectories conforming to the planned fastener extensions.

It has been found that the surgical results of spinal rod implantations critically depend on the bone density of the vertebrae 12 into which the fasteners 16 are to be inserted. Evidently, a fastener 16 cannot be securely anchored in a given vertebra 12 if it is placed in a region of low bone density. It has thus been suggested to take into account bone density data when planning fastener extensions in vertebrae 12.

As an example, US 2005/0101970 A1 teaches an intra-operative surgical planning technique for selecting a most effective screw trajectory out of potential screw trajectories depending upon bone density data. The most effective screw trajectory is the screw trajectory resulting in an optimized screw pullout strength.

It has been found that optimizing the screw pullout strength based on bone density data may lead to better surgical results compared to scenarios in which the bone density is not taken into account. However, there are still anatomical situations in which the surgical results could be improved further.

WO 2020/079598 A discloses a method for optimizing orthopedic spinal implant survival using preoperative finite element analysis combined with intraoperative stress analysis. Based on clinically relevant data, finite element analysis, and corrected values of spinal parameters, an acceptable long-term stress score is determined for an appropriate implant, which is selected from a set of potential implants, such that the shape of the implant minimizes predicted stress values. The clinically relevant data may include bone density measurements. From a preoperative medical image set, values of selected spinal alignment parameters are determined; finite element analysis is performed on potential implants to determine stress values; and a selected implant is digitally positioned in the medical image set to create a virtual bone/implant configuration. After the selected implant is inserted and bent to shape, actual stress values are measured intraoperatively. The process of bending and measuring stress values is repeated until the bone/implant configuration falls within the acceptable long-term stress score range.

US 2019/336179 A1 discloses a system to contour a surgical implant based on intraoperative information.

Agarwal Aakash et al.:"Towards a validated patient-specific computational modeling framework to identify failure regions in traditional growing rods in patients with early onset scoliosis", NASSJ, vol. 5, 1 march 2021, discloses a patient-specific finite element scoliotic model to match the pre-operative scoliotic curve of a patient. A dual stainless-steel traditional growing rod construct was implanted into this scoliotic model and the surgical procedure was simulated to match the post-operative (post-op) scoliotic curve parameters. Muscle stabilization and gravity was simulated through follower load application. Rod distraction magnitudes were chosen based on pre-op to post-op cobb angle correction, and flexion bending load was simulated to identify the high stress regions on the rods.

### Summary

There is a need for a technique for computer-assisted planning of fastener placement in vertebrae that yields an improvement in regard to the surgical results.

According to a first embodiment of the invention, a method is provided of computer-assisted planning of fastener placement in vertebrae that are to be stabilized by a pre-formed spinal rod, wherein two or more fasteners are to couple the rod to the vertebrae. At least one of a target shape of the spine and a shape of the pre-formed rod is defined in patient-specific shape data, a bone density of at least one of the vertebrae is defined in patient-specific bone density data, and patient-specific anatomical data are provided. The method comprises calculating, based on the shape data and the anatomical data, a force distribution along a length of the rod and generating, based on the bone density data of a given vertebra and the calculated force distribution, a planning result for fastener placement.

The pre-forming of the spinal rod may relate to a deviation from a straight (i.e., linear) longitudinal extension. As an example, the pre-forming may result in an S-shape or a portion of an S-shape. The pre-formed rod may have a length that extends over two, three or more vertebrae. In particular, the pre-formed rod may extend over four or more vertebrae.

The planning result may be configured for visualization (e.g., to enable a visual preoperative surgical analysis or to provide visual intra-operative surgical navigation assistance). In some variants, the planning result may be indicative of at least one of a fastener extension in the vertebra, a fastener attachment position along a length of the rod and at least one fastener parameter of a fastener to be inserted into the vertebra.

The planned fastener extension in the vertebra may be defined by one or more of an extension direction, an extension length, an extension endpoint and an extension startpoint. The planned fastener extension may be used for surgical navigation purposes to define a fastener trajectory towards the vertebra that is to receive the fastener.

In some implementations, generating the planning result comprises analyzing a bone density of the given vertebra, as indicated in the bone density data. The analysis may be performed to determine at least one of the fastener extension, the fastener attachment position and the fastener parameter, or another planning result for fastener placement. The corresponding planning result may fulfil at least one predefined stability criterion in view of a force, as indicated by the force distribution, acting on a fastener. The at least one predefined stability criterion may be selected from one or more of a bone stability criterion (e.g., a capability to support a force exerted by a fastener), sagittal balance requirement (e.g., in regard of torsion or gradient of along a length of the rod), a static physiological impact (e.g., upon standing or sitting) and a non-static physiological impact (e.g., upon walking or lifting).

The stability criterion may be defined dependent on the force distribution. The bone stability criterion may be defined based on the force distribution, such as by at least one of a direction and an absolute value of a force vector as indicated in the force distribution. The bone stability criterion defined based on the force distribution may then be evaluated in view of the bone density data. In this regard, the bone stability criterion may be evaluated relative to an average or aggregated bone density in a region of a possible (e.g., iteratively varied) fastener extension and/or a region adjacent to that possible fastener extension. In case it is found that the bone stability criterion is fulfilled, the possible fastener extension may become the planning result.

The patient-specific anatomical data underlying calculation of the force distribution may include one or more geometry-related anatomical parameters and one or more weight-related anatomical parameters. Those two anatomical parameter types may jointly be evaluated to derive a mass distribution of a patient. From the patient's mass distribution, the shape data, one or more optional further parameters (e.g., body mass index, gender, age) and one or more optional stability constraints, the force distribution may be calculated.

The patient-specific shape data define at least one of a target (i.e., planned) shape of the spine and a shape of the pre-formed rod. In some variants, the rod is preformed so as to achieve the target shape of the spine after implantation. The target shape of the spine may be defined first and then translated into a shape of the preformed rod. Pre-forming of the rod may be done manually by the surgeon (e.g., pre-operatively or intra-operatively). Alternatively, pre-forming of the rod may be performed at a manufacturing site (e.g., based on the patient-specific shape data). In such a case, an additive manufacturing technique such as 3D-printing can be used.

The method may comprise generating, based on the planning result, a navigation instruction. Then navigation instruction may, for example, be generated based on at least one of the fastener extension in the vertebra and the fastener attachment position along the rod. Generation of the planning result may involve user input (e.g., a confirmation or modification of an automatically proposed fastener extension or fastener attachment position). One of both of the planning result and the navigation instruction may be output to a user (e.g., visually on a display screen) or to a surgical robot (e.g., as a data set controlling movement of a robotic arm). The planning result may be generated and/or output pre-operatively. The navigation instruction may be generated and/or output intra-operatively.

The navigation instruction may be configured to guide a surgical tool handled by a surgeon or a surgical robot. At least one of a position and an orientation of the surgical tool may be tracked and used for generation of the navigation instruction.

The planning result may be output as a data set or as a set of display instructions. The planning result thus output may comprise the at least one fastener parameter and an indication of the given vertebra that is to receive a fastener having the at least one fastener parameter. The at least one fastener parameter may be indicative of at least one of a fastener length, a fastener diameter, a fastener thread (e.g., a thread pitch), and a fastener type. At least one of the fasteners intended to couple the rod to the vertebrae may comprise a bone screw (e.g., a pedicle screw), a bone peg and a K-wire.

The force distribution calculated based on the shape data and the anatomical data may be indicative of at least one force vector component that extends non-coaxial (e.g., oblique) to at least one of the fastener extension in the vertebra and a length of a fastener when attached to the rod. The force distribution may be indicative of multiple force vectors at two or more different positions along the length of the rod. Each force vector may be indicative of the force exerted on a vertebra by a fastener that is attached at a given position along the length of the rod. The force distribution may be an estimation that is based on the shape data and the anatomical data.

The force distribution may be further calculated based on generic rod data. The generic rod data may be indicative of at least one of a rod length, a rod diameter and a rod material parameter (e.g., indicative of a material stiffness). In some variants, in particular for short rod lengths that are to cover only two or three vertebrae, the rod may have a generic shape along its longitudinal extension.

The patient-specific anatomical data may be indicative of one or more of a pelvic tilt, a sagittal vertical axis translation, a pelvic incidence-lumbar lordosis mismatch, a relation of a C7 plumb line to a pelvic tilt center point, a waist-to-height ratio, a waist-to-hip ratio, a mass distribution along a spine axis, a body-mass-index, a body weight, a pelvic incidence, and a thoracic kyphosis. The anatomical data may be measured or estimated. The anatomical data may at least partially be derived from image data of the patient for whom the planning is performed.

Another embodiment of the invention is defined by a computer program product comprising program code portions that cause a processor to perform the method presented herein when the computer program product is executed by the processor. The processor may be comprised by a local computing system set up in an operating room, by a central server or by cloud computing resources. The computer program product may be stored on a CD-ROM, semiconductor memory, or the computer program product may be provided as a data signal (e.g., when being downloaded from an Internet server).

A further embodiment of the invention is directed at an apparatus for planning of fastener placement in vertebrae that are to be stabilized by a pre-formed spinal rod, wherein two or more fasteners are to couple the rod to the vertebrae. At least one of a target shape of the spine and a shape of the pre-formed rod is defined in patient-specific shape data, a bone density of at least one of the vertebrae is defined in patient-specific bone density data, and patient-specific anatomical data are provided. The apparatus is configured to calculate, based on the shape data and the anatomical data, a force distribution along a length of the rod. The apparatus is further configured to generate, based on the bone density data of a given vertebra and the calculated force distribution, a planning result for fastener placement.

As explained above, the planning result for fastener placement may be indicative of at least one of a fastener extension in the vertebra, a fastener attachment position along a length of the rod and at least one fastener parameter of a fastener to be inserted into the vertebra.

The apparatus is configured to perform the any of the methods and method steps presented herein.

### Brief Description of the Drawings

Further details, advantages and aspects of the present disclosure will become apparent from the following embodiments taken in conjunction with the drawings, wherein:
- Fig. 1: schematically illustrates a spinal rod stabilizing multiple vertebrae;
- Figs. 2A & B: schematically illustrate a surgical planning system with optional imaging capabilities;
- Fig. 3: schematically illustrates a process of obtaining a pre-formed spinal rod;
- Fig. 4: illustrated the process of obtaining bone density data from CT data;
- Figs. 5A - 10: illustrate how exemplary anatomical parameters can be obtained;
- Fig. 11: illustrates a flow diagram of a method implementation of the present disclosure;
- Fig. 12: schematically illustrates a force distribution acting along a length of a spinal rod;
- Fig. 13A & B: schematically illustrate the processing of bone density data;
- Fig. 14: schematically illustrate a surgical navigation scenario that is based on tracking a surgical tool; and
- Fig. 15: schematically illustrates a navigation view in the surgical navigation scenario of Fig. 14.

### Detailed Description

In the following description of exemplary realizations of the present disclosure, the same reference numerals are used to denote the same or similar components.

The realizations described hereinafter pertain to a computer-assisted technique for planning the placement of fasteners 16 in vertebrae 12 that are to be stabilized by a pre-formed spinal rod 10 are (see Fig. 1). While those realizations are primarily described in the context of generating surgical planning information to assist a surgeon guide a surgical tool during a spinal intervention, it will be appreciated that the planning information could alternatively, or additionally, be used to control a surgical robot that operates in a fully automated or semi-automatic manner. As understood herein, a semi-automatic operation includes a scenario in which the handling of a surgical tool by a surgeon is constrained by the surgical robot.

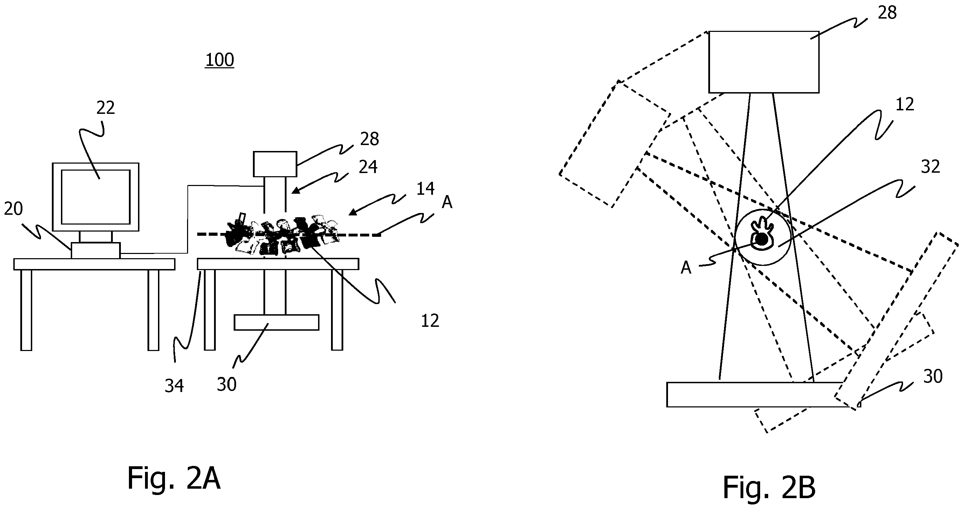

Fig. 2A schematically illustrates an exemplary surgical planning system 100 that is also configured to obtain at least a part of the input data required to generate a planning result for a spinal intervention. In other variants, the input data may be obtained from one or more other systems.

The surgical planning system 100 of Fig. 2A comprises a planning apparatus 20 configured to generate a surgical planning result and an output device 22 that is configured to output the planning result (e.g., for pre-operative information, verification, modification or confirmation purposes, or for intra-operative navigation purposes). Generation of the planning result by the apparatus 20 may include a user interaction (e.g., a verification, modification or confirmation of an automatically generated planning result proposal). In such a case, a user input device such as a keyboard or mouse may be provided (not shown in Fig. 2A)

In Fig. 2A, the planning apparatus 20 is realized as a locally deployed computer system. Alternatively, the apparatus 20 may be realized in the form of a remote server or in the form of cloud computing resources.

In the scenario of Fig. 2A, the output device 22 is a display device configured to visually output the planning result. In other variants, the output device 22 may be configured to (e.g., additionally or alternatively) output one or more of acoustic and haptic information. The output device 22 could be configured as, or comprise, a computer monitor, an augmented reality device (e.g., a head-mounted display, HMD), a loudspeaker, an actuator configured to generate haptically detectable information, or as a combination thereof.

The planning apparatus 10 is configured to generate the planning result from input data Therefore, in an initial stage of the computer-assisted planning technique described herein, the input data have to be obtained by the planning system 100. In the realizations described herein, the input data include at least patient-specific shape data defining a shape of the pre-formed rod 10 or a target shape of a patient's spine 14, patient-specific bone density data defining a bone density of at least one of the vertebrae 12, and patient-specific anatomical data.

In one implementation, at least some of the input data are obtained using surgical imaging techniques. For this reason the surgical planning system 100 of Fig. 2A comprises an imaging apparatus 24 configured to (e.g., pre- or intra-operatively) acquire image data of at least some vertebrae 12 of the patient's spine 14. Those image data will in some variants be used to generate one or more, or all, of the patient-specific shape data, the patient-specific bone density data and the patient-specific anatomical data. In those or other variants, one or more, or all, of the patient-specific shape data, the patient-specific bone density data and the patient-specific anatomical data are generated "off-line", or externally (and, e.g., obtained by the planning system 100 in the form of one or more data sets generated at sites remote from the surgical planning system 100).

In the exemplary scenario illustrated in Fig. 2A, the imaging apparatus 24 of the surgical planning system is a C-arm with cone beam computer tomography (CBCT) imaging capabilities. In other scenarios, the imaging apparatus 24 is a conventional CT scanner that generates slice images, an EOS scanner or an X-ray apparatus. In still further scenarios, the imaging apparatus 24 utilizes a magnet resonance (MR)-based or ultrasound-based imaging technique.

The exemplary CBCT-based imaging apparatus 24 of Fig. 2A comprises a radiation source 28 configured to generate a cone-shaped beam of radiation. Moreover, the imaging apparatus 24 has a flat-panel detector 30 configured to detect the radiation beam projected by the radiation source 28 through one or more of the vertebrae 12. The detector 30 is configured to generate image data representative of two-dimensional projection images of the vertebrae 12. As illustrated in Fig. 2B, such projection images of an imaged volume 32 containing the vertebrae 12 are taken at two more angular orientations of the C-arm relative to an imaginary longitudinal axis A of the vertebrae 12. From the resulting two-dimensional images, in some variants three-dimensional image data of the vertebrae 12 are derived using reconstruction (e.g., back-projection) techniques. The reconstruction may be performed either by the imaging apparatus 24 or by the planning apparatus 20.

Once raw data have been obtained with the aid of one or more of the imaging techniques discussed above, or other data acquisition techniques, the raw data are processed to generate one or more, or all, of the patient-specific shape data, the patient-specific bone density data and the patient-specific anatomical data required for generation of the planning result.

The patient-specific anatomical data are used in combination with the patient-specific shape data to calculate a force distribution at one or more positions along a length of the rod 10. In some implementations, the patient-specific shape date define at least one of a planned, or target, spine shape and an actual or planned rod shape. For example, the patient-specific shape data may define a planned shape of the spine 14 after implantation of the rod 10 and may be used to manufacture the pre-formed rod 10.

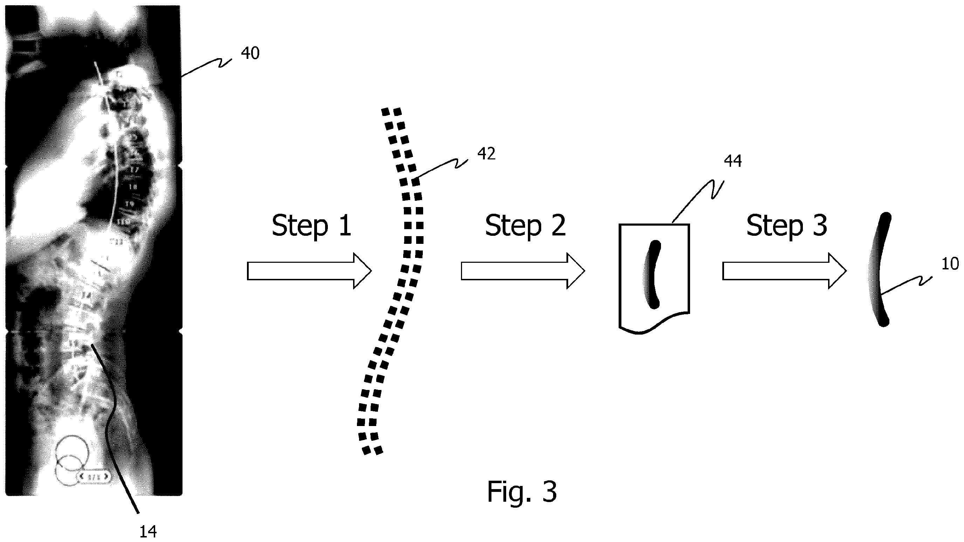

The pre-formed rod 10 may be manufactured in a multi-step procedure, as exemplarily illustrated in Fig. 3. Starting with two-dimensional or three-dimensional image data 40 acquired by the imaging apparatus 24 or obtained otherwise, as schematically shown on the left-hand side of Fig. 3, a user in a first step generates a planned (or target) shape model 42 of at least a portion of the spine 14 (i.e., is indicative of a spine shape that should result from implantation of the rod 10). The planned (or target) shape model 42 is in some variants defined by the user via the planning apparatus 20 or another computer system. To this end, the image data 40 are visualized (e.g., on the output device 22) and the user is given the opportunity to define the planned shape model 42 (e.g., relative to the visualized image data 40).

In a second step that is illustrated in the center of Fig. 3, the planned (or target) shape model 42 thus defined is translated into a data set 44 indicative of the patient-specific shape data. In the present example, the data set 44 is indicative of the planned rod shape in terms of its longitudinal extension (that will deviate from a straight, or linear, extension). The rod shape will typically include one or more bends (e.g., in the form of an "S" or a portion of an "S"). The data set 44 may describe a computer aided design (CAD) model of the preformed rod 10. In a first implementation, a user-indicated portion of the planned (or target) shape model 42 (e.g., between two user-indicated vertebrae 12 such as L1 and L5, in the conventional notation) is translated into the data set 44. In a more refined second implementation, the user may (e.g., additionally) define further shape constraints, such as a rod diameter, that will be entered in the data set 44. In a third implementation, those shape constraints are pre-defined. In typical realizations, the rod 10 will extend over, and need to be attached to, three, four or more vertebrae.

In a third step illustrated on the right-hand side of Fig. 3, the actual rod 10 is manufactured based on the data set 44 using, for example, an additive manufacturing, a rod bending machine or any other manufacturing/shaping technique.

In an alternative variant, the shape of the rod 10 is manually defined by a user (such as a surgeon) by bending a straight or generically pre-bent rod with a bending tool according the patient's needs. The shape of the actual rod 10 is then translated into a data set indicative of the patient-specific shape data by scanning the user-bent rod 10 using a laser scanner, or otherwise.

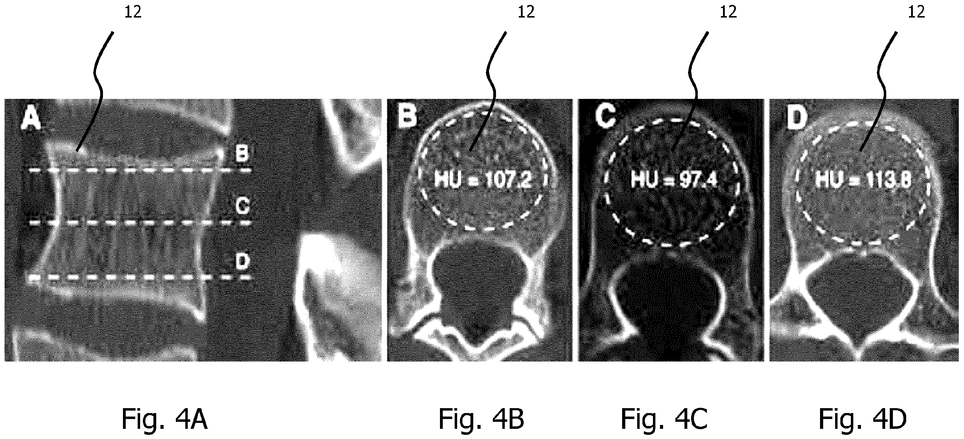

The patient-specific bone density data may likewise be derived from image data obtained using one or more of the imaging techniques discussed above, or other data acquisition techniques. In some variants, the patient-specific bone density data are derived from three-dimensional CT image data as illustrated in Figs. 4A to 4D.

Fig. 4A shows a view of a particular vertebra 12 taken along the saggital plane and indicates the location of three transverse planes B, C, D that are illustrated in Figs. 4B, 4C and 4D, respectively. Figs. 4B, 4C and 4D visualize the inner structures of the vertebra 12 of Fig. 4A and are processed further to derive volumetric (i.e., three-dimensional) bone density data for the vertebra 12. Such processing can be based on Hounsfield units (HUs) which are taken as, or converted into, actual bone density data. In Figs. 4B, 4C and 4D, mean HUs are indicated for a certain cross-sectional area.

In some implementations, a dedicated bone density data set is generated per vertebra 12 of interest. In such implementations, vertebra segmentation techniques are applied on the image data.

At least some of patient-specific anatomical data may be acquired from image data obtained using one or more of the imaging techniques discussed above, in particular using an EOS scanner. Of course, other data acquisition techniques can be used as well.

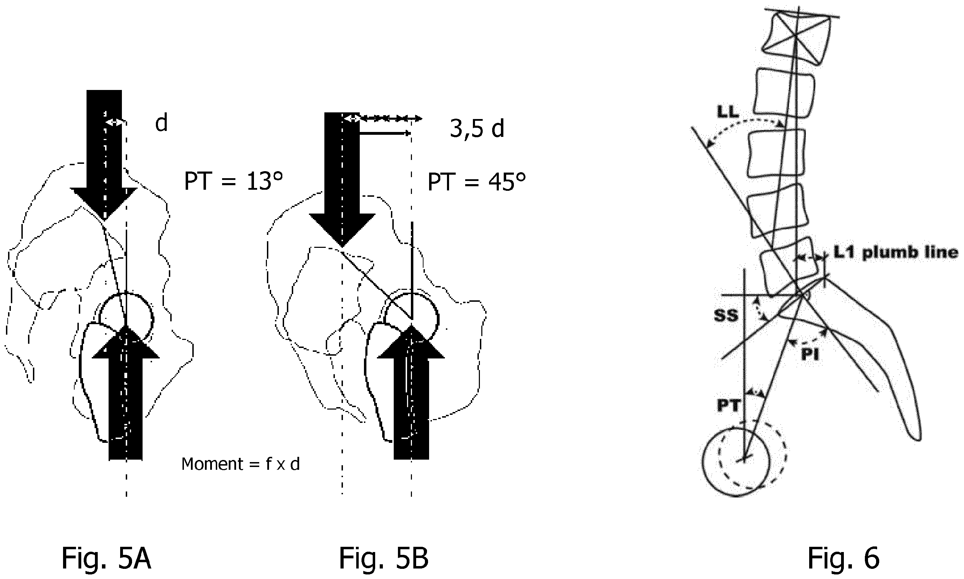

Exemplary patient-specific anatomical parameters that may be comprised by the anatomical data will now be described with reference to Figs. 5A to 10.

Figs. 5A and 5B illustrate different patient-specific pelvic tilts (PTs) of 13° and 45°, respectively. As is well known in the art, a larger pelvic tilt requires a higher force for lumbar stabilization. The reason is that for a given weight f, the moment required for stabilization increases with the distance d between the two lines illustrated in each of Figs. 5A and 5B. In other words, higher forces will act on the rod 10 with increasing pelvic tilt, or put differently, will be transferred by the fasteners 16 (that are attached to the rod 10) on the vertebrae 12.

With reference to Fig. 6, pelvic incidence-lumbar lordosis (PI-LL) mismatch is a patient-specific anatomical parameter that can be used to calculate a force vectors within the lumbar section along the length of the pre-formed rod 10, also depending on the pelvic tilt. In Fig. 6, SS indicates a sacral slope.

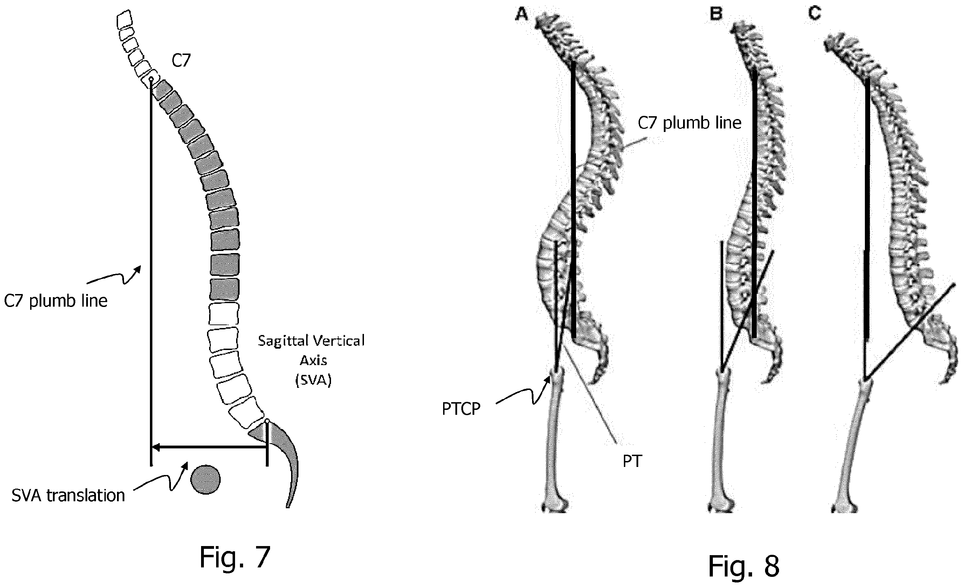

With reference to Fig. 7, saggital vertical axis (SVA) translation is a further patient-specific anatomical parameter that can be used in some realizations of the present disclosure for calculating the force distribution on the rod 10. SVA permits an estimation of force vectors (in terms of force strengths and force directions) acting on the pre-formed rod 10 in the lumbar section. In a similar manner, the relation between the C7 plumb line and the pelvic tilt center point (PTCP) as illustrated in Fig. 8 for three scenarios A, B and C has a significant influence in the determination of force strengths and force directions acting on the pre-formed rod 10 along its longitudinal extension.

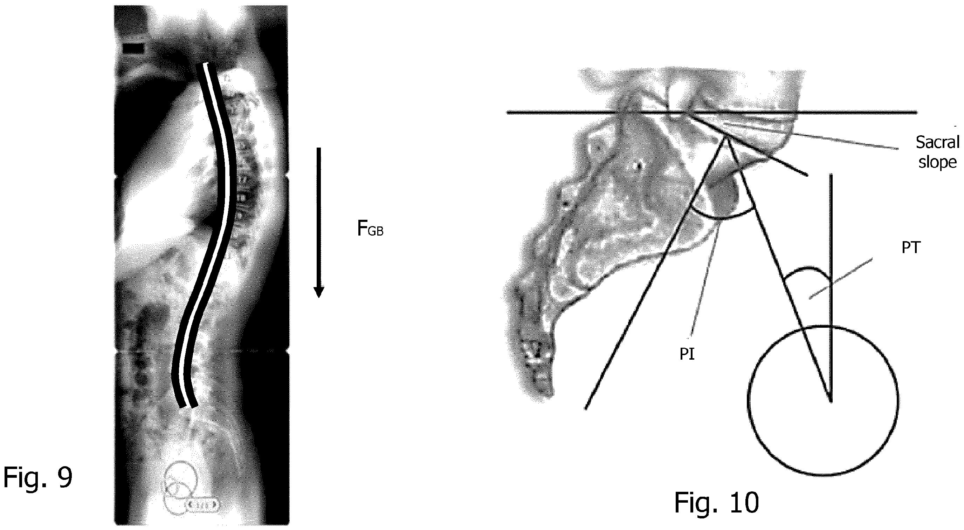

With reference to Fig. 9, the resulting force F_{GB} from a global balancing analysis is shown. F_{GB} is calculated from a physiological shape of the patient anatomy and in particular the spine (i.e., a geometry-related anatomical parameter), the mass distribution of the patient (i.e., a weight-related anatomical parameter), and optional further parameters, such as gender, age and body mass index.

Referring now to Fig. 10, the pelvic incidence (PI) and sacral slope are also patient-specific anatomical parameters that influence global balancing and, thus, the forces acting on the pre-formed rod 10 along its extension, albeit in a secondary order of magnitude. Similarly, thoracic kyphosis can be considered as a patient-specific anatomic parameter for calculating the forces acting on the pre-formed rod 10.

Further patient-specific anatomic data include parameters such as a waist-to-height ratio, a waist-to-hip ratio, a mass distribution along a spine axis, a body-mass-index, and a body weight. Evidently, one, two or more of the above patient-specific anatomic parameters, and other patient-specific anatomic parameters, may be used to define patient-specific anatomical data as input for the planning apparatus 20.

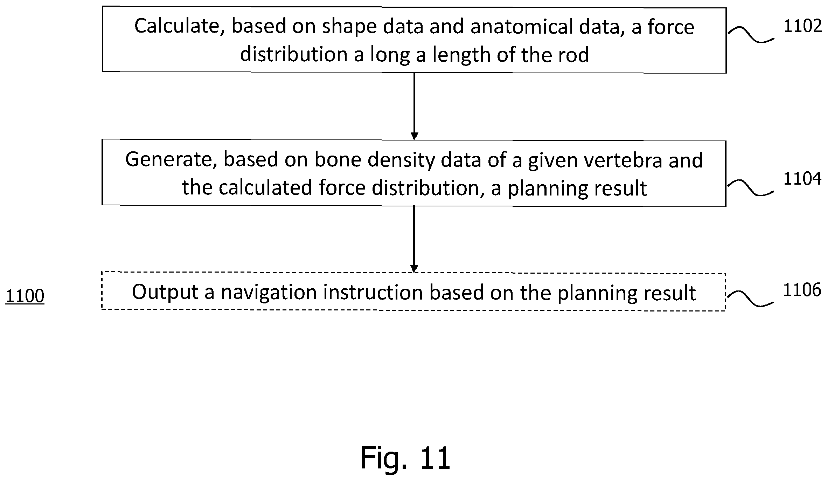

Having now discussed how exemplary patient-specific anatomical data, patient-specific shape data and patient-specific bone density data can be obtained, the processing of those input data in the context of surgical planning will now be explained with reference to the flow diagram 1100 of Fig. 11. The flow diagram 1100 illustrates a method implementation of the present disclosure as performed by the planning apparatus 20 for planning fastener placement in multiple vertebrae 12 that are to be stabilized by the pre-formed rod 10, as illustrated in Fig. 1.

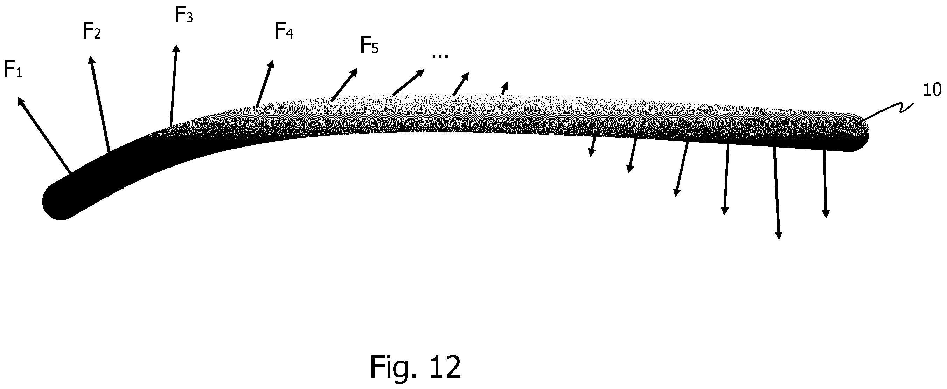

In step 1102, the planning apparatus 20 calculates, based on the patient-specific shape data and one or more of patient-specific anatomical parameters (i.e., anatomical data), a force distribution at one or more along a length of the rod 10, as schematically illustrated in Fig. 12. For calculating the force distribution, one or more geometry-related anatomical parameters (such as one or more of PT, SVA translation, PI-LL mismatch and PI) and one or more weight-related anatomical parameters (such as one or more of a mass distribution along a spine axis, a body-mass-index, and a body weight) are jointly evaluated taking into account the shape data that define at least one of a target shape of the spine 14 and a shape of the pre-formed rod 10 (see also the discussion of Fig. 9 above). In some variants, the force distribution is also calculated based on generic rod data. The generic rod data are indicative of at least one of a rod length, a rod diameter and a rod material parameter. The generic rod data may at least partially be comprised by the patient-specific shape data as a rod length or rod diameter may have been selected in a patient-specific manner (as described above with reference to Fig. 3).

The force distribution can be calculated for a series of two or more positions along the length of the rod 10 and possibly using the information illustrated in Fig. 9. At each position considered, a force vector jointly defined by a force value, or force strength, and a force direction may be calculated. As such, the force distribution is in some variants indicative of force vectors at different positions along a length of the rod 10. Each force vector may be indicative of the force that will be transferred by a fastener 16 (when hypothetically attached to the rod 10 at a given position along its length) on the associated vertebra 12

As very schematically shown in Fig. 12, the force vectors F₁, F₂, F₃, etc. will typically not be plain "pullout force vectors" facing, from the perspective of possible fastener attachment positions at the rod 10, away from the vertebra 12 in which a particular fastener 16 is to be inserted. Rather, the forces may also include compression forces that are directed towards a particular vertebra 12, or may at least include such force vector components. Other force vector components may be directed obliquely (i.e., may extend non-coaxially) to at least one of a fastener extension in the particular vertebra 12 and a length of the fastener 16 when attached to the rod 10.

In step 1104 of Fig. 11, the planning apparatus 20 generates, based on the bone density data of a given vertebra 12 (see Fig. 4) and the calculated force distribution (see Fig. 12), a planning result. The planning result thus calculated may be presented on the output device 22 (see Fig. 2A) to a user for the purpose of information, verification, confirmation or modification. Moreover, as will be discussed in greater detail below, the planning result can also form the basis of surgical navigation instructions for guiding either a surgeon or a surgical robot.

In some implementations, the planning result is indicative of a fastener extension in a particular vertebra 12. The planned fastener extension may be defined by one or more of an extension direction, an extension length, an extension endpoint in the vertebra 12 and an extension startpoint on a surface of the vertebra 12. The planned fastener extension in the vertebra 12 can be used for surgical navigation purposes to define a fastener trajectory towards that vertebra 12. The fastener trajectory towards the vertebra 12 will typically be defined by a line co-linear with the fastener extension in the vertebra 12.

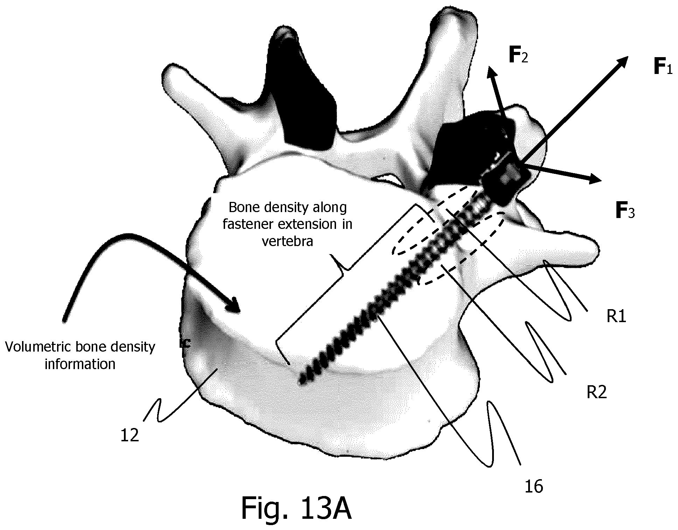

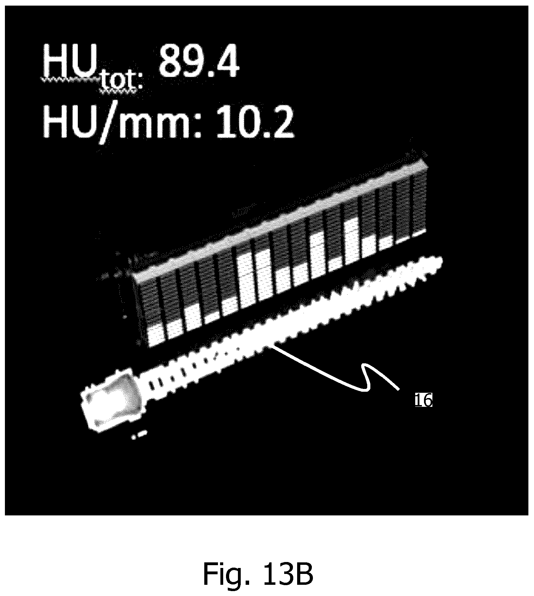

It is generally desirable to select the fastener extension in the vertebra 12 such that the fastener 16 is securely anchored therein. As such, the fastener 16 should extend in and/or adjacent to a region of high bone density. Selecting, however, fastener extension solely based on the bone density data such that, for example, the pullout force is optimized neglects the finding that the forces transmitted on a given fastener 16 by the rod 10 (that connects multiple such fasteners 16) will have force vector components that extend obliquely to the extension of the given fastener 16 in the vertebra 12 or even in a direction opposite to a pullout direction, as illustrated in Fig. 12. It is therefore proposed that the fastener extension in a given vertebra 12 be selected in step 1104 not only dependent on bone density data of that vertebra 12, but also based on the force distribution calculated in step 1102, as will now be explained with reference to Figs. 13A and 13B.

Fig. 13A illustrates a vertebra 12 for which volumetric bone density information has been obtained, for example as explained above with reference to Fig. 4. Using this volumetric bone density information, the bone density and bone density variation along any selected fastener extension in the vertebra 12 can be evaluated. The fastener extension may for this purpose be defined by a cylindrical volume within the bone density volume derived for the vertebra 12. The cylindrical volume, in turn, may in some implementation depend on one or more fastener parameters, such as fastener diameter. As illustrated in Fig. 13B, for any fastener extension, an aggregated bone density (HUₜₒₜ) and an average bone density per length unit (HU/mm) along the fastener extension can be calculated. Moreover, in addition or as an alternative to the bone density and bone density variation along a selected fastener extension, the bone density in a region adjacent to the fastener extension can be evaluated. Such an adjacent region may be required to support significant force vector components that extend non-coaxially relative to the fastener extension.

Consequently, the bone density data may be evaluated to determine if a predetermined bone stability criterion is fulfilled. The bone stability criterion may be defined based on the force distribution.

Assume, for example, that the force distribution indicates for a given (e.g., user-selected, iteratively varied or automatically planned) fastener attachment position along a length of the rod 10 (not illustrated in Fig. 13A) that the force vector acting at this position on the fastener 16 is directed substantially co-axially along a length of the fastener 16 away from the associated vertebra 12, see force vector F₁ in Fig. 13A. In such a situation, it may generally be sufficient to plan the fastener extension in that vertebra 12 by the apparatus 20 such that the aggregated bone density along the fastener extension in the vertebra 12 (see Fig. 13B) is maximized or at least lies above a selected threshold. The threshold may constitute a bone stability criterion and be selected based on an absolute value, or strength, of the force vector or of a "pullout" vector component thereof.

For generating a proper fastener extension in a given vertebra 12, and for a given force vector F₁, the planning apparatus 20 may thus start with evaluating a predetermined fastener extension (e.g., that extends perpendicularly from and, optionally, lies within a plane of the pre-formed rod 10). The fastener extension may then (e.g., iteratively) be modified (e.g., with respect to its angular extension relative to the rod 10) to maximize the aggregated bone density along the fastener extension or until the aggregated fastener extension lies above the selected threshold.

If, on the other hand, the force distribution indicates for a given (e.g., user-selected, iteratively varied or automatically planned) fastener attachment position along a length of the rod 10 that the force vector acting at this position on the fastener 16 has a significant vector component obliquely to a length of the fastener 16 (see force vectors F₂ and F₃ in Fig. 13A), then it will be important that there is a sufficiently high bone density in a region at which the force will mainly be supported by bone. Such scenarios are illustrated by region R1 for force vector F₂ and region R3 for force vector F₃ in Fig. 13B, which are located adjacent to the fastener extension in the vertebra 12. Compared to the situation of force vector F₁, there may be a lower force vector component in a fastener pullout direction, so that planning the fastener extension such that the aggregated bone density is optimized may still lead to bone fragmentation in case of a low bone density in a local vertebra region that needs to support a significant amount of force, such as region R1 for force vector F₂ and region R2 for force vector F₃ in Fig. 13A. One or both of the locations and sizes of the a region such as region R1 or R2 may be defined, as a bone stability criterion, based on the force distribution (e.g., based on a force vector direction). The (e.g., average or aggregated) bone density in the region may in some variants be subjected to threshold decision similar to the scenario discussed above with reference to force vector F₁.

For generating a proper fastener extension in a given vertebra 12 and for a given force vector F₂ or F₃, the planning apparatus 20 may thus start with evaluating a predetermined fastener extension (e.g., that extends perpendicularly from the rod 10 and, optionally, lies within a plane of the pre-formed rod 10). The fastener extension may then (e.g., iteratively) be modified (e.g., with respect to its angular extension relatively to the rod 10 and/or with respect to a fastener attachment position along a length of the rod 10) until the fastener extension is found to be adjacent a region of high bone density at the required position.

In some implementations, the planning result is - additionally to the fastener extension, or only - indicative of a fastener attachment position along a length of the rod 10. As will be appreciated from Fig. 12, the attachment position of a fastener 16 along the length of the rod 10 has an influence on both the force transmitted by the fastener 16 into a given vertebra 12 and the extension of that fastener 16 in that vertebra 12. Therefore, within specific bounds, also the fastener attachment position along the length of the rod 10 for a given vertebra 12 can be planned by the planning apparatus 20, taking into account the bone density data and the force distribution, such that certain surgical requirements will be met.

In the example of Fig. 13A and for force vector F₂ or F₃, the fastener attachment position on the rod can be selected such that a fastener extension startpoint on a surface of the vertebra 12 will be adjacent to a region R1 or R2, respectively, of a high bone density at the required side of the fastener 16. Evidently, the fastener extension (e.g., in terms of a extension direction as seen from the rod 10) and the fastener attachment position along the length of the rod can in same situations be planned (e.g., optimized) simultaneously so as to achieve the best surgical outcome.

In some implementations, the planning result is - for example in addition to one of both of the fastener extension and fastener attachment position - indicative of at least one fastener parameter of a fastener 16 that is to be inserted into a given vertebra 12. The at least one fastener parameter may be indicative of at least one of a fastener length, a fastener diameter, a fastener thread (e.g., thread type, thread pith, thread length), and a fastener type. The types of fasteners 16 intended to couple the rod to the vertebrae may comprise at least one of a bone screw (e.g., a pedicle screw, see Figs. 1, 13A and 13B), a bone peg and a K-wire. If for example, it is found by the planning apparatus 20 that the bone density of a given vertebra 12 is low in view of a high amount of force to be accommodated in that vertebra 12, the planning result may indicate one or both of a larger fastener diameter and a longer fastener length compared to a scenario of higher bone density or lower force.

The planning result may comprise, in addition to the at least one fastener parameter, and an indication of the given vertebra that is to receive a fastener having the at least one fastener parameter. The vertebra may be indicated visually (e.g., in the context of a visualization of CT data) or using a conventional (such as L1, L2, etc.) or proprietary notation.

As has become apparent from the above, generating the planning result may include analyzing a bone density of a given vertebra 12, as indicated in the bone density data, to determine at least one of the fastener extension, the fastener attachment position and the fastener parameter such that at least one predefined stability criterion is fulfilled in view of a force, as indicated by the force distribution, acting on a fastener 16. The at least one predefined stability criterion may be a bone stability criterion, as explained above. In other scenarios, the predefined stability criterion may be at least one of a sagittal balance requirement, a static physiological impact and a non-static physiological impact.

In some variants, the bone density data are correlated with the calculated force distribution. In bone positions where high forces will be acting there is a need for a high (e.g., maximal) bone density. In bone positions where low forces will be acting no high bone density is required. Threshold decisions may be applied for assessing forces as high/low and bone densities as high/low (of course, the present disclosure is not limited to such binary decisions but could also be implemented with a higher granularity). The resulting force distribution, or force accommodation requirement, can be derived from the one or more stability criteria. As an example, the saggital balance requirement influences the resulting force distribution in that a saggital balance of the spine has to be (re-)established.

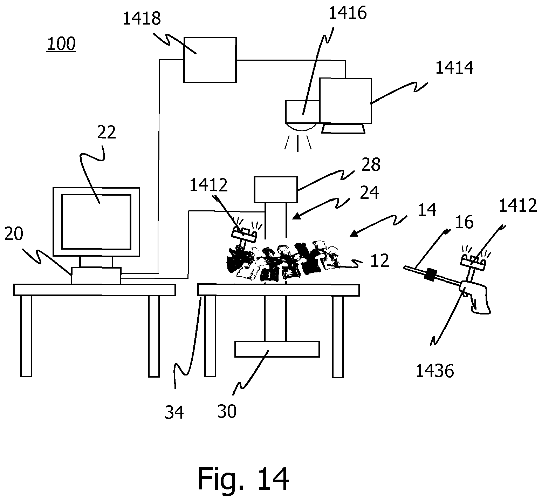

In an optional step 1106, a navigation instruction is generated based on the planning result, and the navigation instruction is output to a surgeon or a surgical robot. Generation of the navigation instruction will in some scenarios require a tracking of a surgical tool operated by the surgeon or surgical robot. To this end, as illustrated in Fig. 14, the surgical system 100 may comprise optical surgical tracking components including one or more optical trackers 1412, a camera 1414, an (optional) source of electromagnetic radiation 1416, and a tracking controller 1418. Of course, the tracking components may in other cases be selected for electromagnetic tracking or inertial tracking.

The source of electromagnetic radiation 1416 is configured to emit at least one of infrared light and visible light. The source of electromagnetic radiation 1416 may specifically be configured to flood the entire surgical site with electromagnetic radiation. Each of the one or more trackers 1412 comprises three or more reflectors (e.g., spherically shaped bodies) that reflect the electromagnetic radiation emitted by the source of electromagnetic radiation 1416. As such, the one or more trackers 1412 are configured as so-called passive trackers. In other variants, at least one of the one or more trackers 1412 may be realized as an active device configured to emit electromagnetic radiation. As an example, each active tracker 1412 may comprise three or more light emitting diodes (LEDs) emitting electromagnetic radiation in the infrared or visible spectrum. If all the trackers 1412 are configured as active devices, the source of electromagnetic radiation 1416 may in some variants be omitted.

The camera 1414 has at least one image sensor, such as a charged couple device (CCD) or a complementary metal-oxide-semiconductor sensor (CMOS). The image sensor is configured to detect the electromagnetic radiation reflected (or emitted) by the one or more trackers 1412. In some variants, the camera 1414 may have multiple image sensors. In particular, the camera 1414 may be a stereo camera with at least two image sensors.

The tracking controller 1418 is configured to process the image data generated by the camera 1414 and to calculate the position and orientation of the one or more trackers 1412 in a tracking coordinate system. This calculation is typically performed in 5 or 6 DOF. The tracking coordinate system may have a rigid relationship relative to the camera 1414 and may in particular be centred in a centre of the camera 1414.

In the exemplary scenario illustrated in Fig. 14, a dedicated tracker 1412 is provided for a surgical tool 1436 such as a surgical screwdriver configured to screw a fastener 16 into a vertebra 12. In other variants, the surgical tool 1436 is a surgical drill configured to drill a hole into a vertebra 12 for accommodating the fastener 16.

As shown in Fig. 14, the planning apparatus 20 may be communicatively coupled with the tracking controller 1418. In some variants, the planning apparatus 20 jointly processes the planning result and at least one of position and orientation information as received from the tracking controller 18 for intra-operatively generating a navigation instruction. Since the planning result may be generated pre-operatively, the planning result may in other variants be transferred to the tracking controller 1418 or a further device coupled to the tracking controller 1418 (e.g., via a data carrier or communication network) for generation of the navigation instruction.

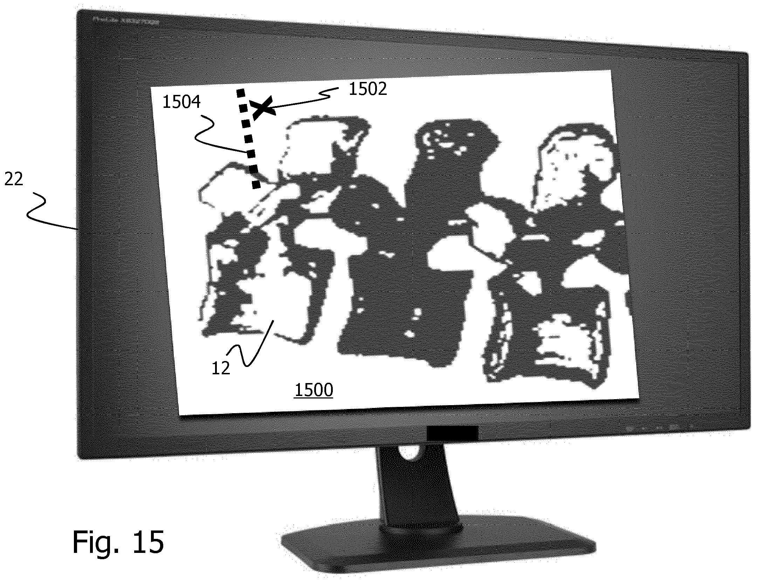

Fig. 15 illustrates an exemplary navigation view 1500 displayed by the output device 22. The navigation view visualizes pre-operatively or intra-operatively acquired image data of the vertebrae 12 (e.g., as taken by the imaging device 24). Those image data have been registered relative to the tracking coordinate system so that at least one of the position and orientation of the tracked surgical tool 1436 or other tracked entity is known relative to the image data.

The navigation instruction in the navigation view 1500 is representative of a current tip position 1502 of the tracked surgical tool 1436 (or of a fastener attached to the surgical tool 1436) relative to a planned fastener trajectory 1504 as derived from the planned fastener extension in the vertebra 12 to be surgically treated. In other scenarios, a tool trajectory may be visualized instead of the tool tip.

As has been explained above, the planned fastener trajectory 1504 may be derived by extrapolating the planned fastener extension outside the particular vertebra 12 that is to receive the fastener 12. The navigation instruction, when output to the surgeon, will cause the surgeon to attempt to align the actual tool tip or trajectory with the planned fastener trajectory. In the scenario illustrated in Fig. 15, the surgeon will thus need to move the tool tip to the left to reach the planned tool trajectory. If the surgeon is replaced, or assisted, by a surgical robot, the navigation instruction is output as a data set to the surgical robot for control or verification purposes.

Once all the required bone fasteners 16 have been implanted in the vertebrae 12 in accordance with the respectively associated planning result (e.g., a fastener extension), the pre-formed rod 10 is coupled to the fastener heads 16A, as illustrated in Fig. 1. In some variants, a tracker 1412 is temporarily attached to the rod 10 so that rod placement can also be guided (e.g., in a navigation view on the displayed by the output device 22) in accordance with a planned rod position.

If the vertebrae 12 are tracked also (either individually or collectively), any movement of the one or more tracked vertebrae 12 will be detected by the tracking controller 1418 (in 5 or 6 DOF). The movement, which can involve one or both of a rotation and a translation, can then be considered in real-time upon generating the navigation instruction by, for example, updating a visual representation of the vertebrae 12 in the navigation view 1500.

As has become apparent from the above description of exemplary realizations of the present disclosure, the technique presented herein improves surgical planning of fastener placement by not merely relying on bone density data, but additionally considering an estimated force distribution in bone. It will be apparent that the exemplary realizations discussed above can be modified in many ways.

## Claims

1. A method of computer-assisted planning of fastener placement in vertebrae (12) that are to be stabilized by a pre-formed spinal rod (10), wherein two or more fasteners (16) are to couple the rod (10) to the vertebrae (12), wherein at least one of a target shape of the spine (14) and a shape of the preformed rod (10) is defined in patient-specific shape data, a bone density of at least one of the vertebrae (12) is defined in patient-specific bone density data, and wherein patient-specific anatomical data are provided, the method being performed by an apparatus (20) and comprising:
calculating (1102), based on the shape data and the anatomical data, a force distribution along a length of the rod (10); and
generating (1104), based on the bone density data of a given vertebra (12) and the calculated force distribution, a planning result for fastener placement.

2. The method of claim 1, wherein
the planning result is indicative of at least one of a fastener extension in the vertebra (12), a fastener attachment position along a length of the rod (10) and at least one fastener parameter of a fastener (16) to be inserted into the vertebra (12).

3. The method of claim 1 or 2, wherein
generating (1104) the planning result comprises analyzing a bone density of the given vertebra (12), as indicated in the bone density data, wherein the planning result fulfils at least one predefined stability criterion in view of a force, as indicated by the force distribution, acting on a fastener (16).

4. The method of claim 3, wherein
the at least one predefined stability criterion is selected from one or more of a bone stability criterion, a sagittal balance requirement, a static physiological impact and a non-static physiological impact.

5. The method of any of the preceding claims, wherein
the patient-specific anatomical data underlying calculation of the force distribution include one or more geometry-related anatomical parameters and one or more weight-related anatomical parameters.

6. The method of any of the preceding claims, comprising
generating (1106) a navigation instruction based on the planning result.

7. The method of at least claim 2, comprising
outputting the planning result that comprises at least one fastener parameter and an indication of the given vertebra that is to receive a fastener (16) having the at least one fastener parameter.

8. The method of at least one of claims 2 and 7, wherein
the at least one fastener parameter is indicative of at least one of a fastener length, a fastener diameter, a fastener thread, and a fastener type.

9. The method of any of the preceding claims, wherein
at least one of the fasteners (16) comprises a bone screw, a bone peg and a K-wire.

10. The method of any of the preceding claims, wherein
the force distribution is indicative of at least one force vector component that extends non-coaxial to at least one of the fastener extension in the vertebra (12) and a length of a fastener (16) when attached to the rod (10).

11. The method of any of the preceding claims, wherein
the force distribution is indicative of force vectors at different positions along a length of the rod (10).

12. The method of any of the preceding claims, wherein
the force distribution is further calculated based on generic rod data.

13. The method of claim 12, wherein
the generic rod data are indicative of at least one of a rod length, a rod diameter and a rod material parameter.

14. The method of any of the preceding claims, wherein
the anatomical data are indicative of one or more of a pelvic tilt, a sagittal vertical axis translation, a pelvic incidence-lumbar lordosis mismatch, a relation of a C7 plumb line to a pelvic tilt centre point, a waist-to-height ratio, a waist-to-hip ratio, a mass distribution along a spine axis, a body-mass-index, a body weight, a pelvic incidence, and a thoracic kyphosis.

15. A computer program product comprising program code portions that cause a processor to perform the method of any of the preceding claims when the computer program product is executed by the processor.

16. An apparatus (20) for planning of fastener placement in vertebrae (12) that are to be stabilized by a pre-formed spinal rod (10), wherein two or more fasteners (16) are to couple the rod (10) to the vertebrae (12), wherein at least one of a target shape of the spine (14) and a shape of the rod (10) is defined in patient-specific shape data, a bone density of at least one of the vertebrae (12) is defined in patient-specific bone density data, and wherein patient-specific anatomical data are provided, the apparatus being configured to:
calculate (1102), based on the shape data and the anatomical data, a force distribution along a length of the rod (10); and
generate (1104), based on the bone density data of a given vertebra (12) and the calculated force distribution, a planning result for fastener placement.

17. The apparatus (20) of claim 16, configured to perform the method of any of claims 2 to 14.

## Patentansprüche

1. Verfahren zur computergestützten Planung der Platzierung von Befestigungselementen in Wirbeln (12), die durch einen vorgeformten Wirbelsäulenstab (10) stabilisiert werden sollen, wobei zwei oder mehr Befestigungselemente (16) den Stab (10) an die Wirbel (12) koppeln sollen, wobei mindestens eine von einer Zielform der Wirbelsäule (14) und einer Form des vorgeformten Stabes (10) in patientenspezifischen Formdaten definiert ist, eine Knochendichte von mindestens einem der Wirbel (12) in patientenspezifischen Knochendichtedaten definiert ist, und wobei patientenspezifische anatomische Daten bereitgestellt werden, wobei das Verfahren durch eine Vorrichtung (20) durchgeführt wird und umfasst:
Berechnen (1102) einer Kraftverteilung entlang einer Länge des Stabes (10), basierend auf den Formdaten und den anatomischen Daten; und
Erzeugen (1104) eines Planungsergebnisses für die Platzierung von Befestigungselementen, basierend auf den Knochendichtedaten eines bestimmten Wirbels (12) und der berechneten Kraftverteilung.

2. Verfahren nach Anspruch 1, wobei
das Planungsergebnis mindestens eines angibt aus einer Befestigungselement-Erstreckung in dem Wirbel (12), einer Befestigungselement-Anbringungsposition entlang einer Länge des Stabes (10) und mindestens einem Befestigungselement-Parameter eines in den Wirbel (12) einzubringenden Befestigungselements (16).

3. Verfahren nach Anspruch 1 oder 2, wobei
das Erzeugen (1104) des Planungsergebnisses ein Analysieren einer Knochendichte des bestimmten Wirbels (12), wie sie in den Knochendichtedaten angegeben ist, umfasst, wobei das Planungsergebnis mindestens ein vordefiniertes Stabilitätskriterium im Hinblick auf eine Kraft, wie sie durch die Kraftverteilung angegeben ist, die auf ein Befestigungselement (16) wirkt, erfüllt.

4. Das Verfahren nach Anspruch 3, wobei
das mindestens eine vordefinierte Stabilitätskriterium aus einem oder mehreren von einem Knochenstabilitätskriterium, einer sagittalen Gleichgewichtsanforderung, einem statischen physiologischen Einfluss und einem nicht-statischen physiologischen Einfluss ausgewählt ist.

5. Verfahren nach einem der vorhergehenden Ansprüche, wobei
die der Berechnung der Kraftverteilung zugrundeliegenden patientenspezifischen anatomischen Daten einen oder mehrere geometriebezogene anatomische Parameter und einen oder mehrere gewichtsbezogene anatomische Parameter umfassen.

6. Verfahren nach einem der vorhergehenden Ansprüche, umfassend
Erzeugen (1106) einer Navigationsanweisung auf der Grundlage des Planungsergebnisses.

7. Verfahren nach zumindest Anspruch 2, umfassend
Ausgeben des Planungsergebnisses, das mindestens einen Befestigungselement-Parameter und eine Angabe des bestimmten Wirbels umfasst, der ein Befestigungselement (16) mit dem mindestens einen Befestigungselement-Parameter aufnehmen soll.

8. Verfahren nach zumindest einem der Ansprüche 2 und 7, wobei
der mindestens eine Befestigungselement-Parameter zumindest eines aus einer Befestigungselement-Länge, einem Befestigungselement-Durchmesser, einem Befestigungselement-Gewinde, und einem Befestigungselement-Typ angibt.

9. Verfahren nach einem der vorhergehenden Ansprüche, wobei
mindestens eines der Befestigungselemente (16) eine Knochenschraube, einen Knochenpflock und einen K-Draht umfasst.

10. Verfahren nach einem der vorhergehenden Ansprüche, wobei
die Kraftverteilung mindestens eine Kraftvektorkomponente angibt, die sich nicht-koaxial zu mindestens einem von der Befestigungselement-Erstreckung in dem Wirbel (12) und einer Länge eines Befestigungselements (16) erstreckt, wenn es an dem Stab (10) befestigt ist.

11. Verfahren nach einem der vorhergehenden Ansprüche, wobei
die Kraftverteilung Kraftvektoren an verschiedenen Positionen entlang einer Länge des Stabs (10) angibt.

12. Das Verfahren nach einem der vorhergehenden Ansprüche, wobei
die Kraftverteilung ferner basierend auf generischen Stabdaten berechnet wird.

13. Verfahren nach Anspruch 12, wobei
die generischen Stabdaten mindestens eines aus einer Stablänge, einem Stabdurchmesser und einem Stabmaterial anzeigen.

14. Verfahren nach einem der vorhergehenden Ansprüche, wobei
die anatomischen Daten eine oder mehrere angeben aus einer Beckenkippung, einer Translation der vertikalen Sagittalachse, einer Fehlanpassung von Beckeninkzidenz und Lendenlordose, einer Beziehung zwischen einer C7-Lotlinie und einem Beckenkippungsmittelpunkt, einem Verhältnis von Taille zu Körpergröße, einem Verhältnis von Taille zu Hüfte, einer Massenverteilung entlang einer Wirbelsäulenachse, einem Body-Mass-Index, einem Körpergewicht, einer Beckeninkzidenz und einer Thoraxkyphose.

15. Computerprogrammprodukt umfassend Programmcodeabschnitte, die einen Prozessor veranlassen, das Verfahren nach einem der vorangehenden Ansprüche durchzuführen, wenn das Computerprogrammprodukt von dem Prozessor ausgeführt wird.

16. Vorrichtung (20) zur Planung der Platzierung von Befestigungselementen in Wirbeln (12), die durch einen vorgeformten Wirbelsäulenstab (10) stabilisiert werden sollen, wobei zwei oder mehr Befestigungselemente (16) den Stab (10) an die Wirbel (12) koppeln sollen, wobei mindestens eine von einer Zielform der Wirbelsäule (14) und einer Form des Stabes (10) in patientenspezifischen Formdaten definiert ist, eine Knochendichte von mindestens einem der Wirbel (12) in patientenspezifischen Knochendichtedaten definiert ist, und wobei patientenspezifische anatomische Daten bereitgestellt werden, wobei die Vorrichtung eingerichtet ist zum:
Berechnen (1102) einer Kraftverteilung entlang einer Länge des Stabes (10), basierend auf den Formdaten und den anatomischen Daten; und
Erzeugen (1104) eines Planungsergebnisses für die Platzierung von Befestigungselementen, basierend auf den Knochendichtedaten eines bestimmten Wirbels (12) und der berechneten Kraftverteilung.

17. Vorrichtung (20) nach Anspruch 16, die dazu eingerichtet ist, das Verfahren nach einem der Ansprüche 2 bis 14 durchzuführen.

## Revendications

1. Procédé de planification assisté par ordinateur de positionnement de fixations dans des vertèbres (12) qui doivent être stabilisées par une tige rachidienne (10) préformée, dans lequel au moins deux fixations (16) doivent accoupler la tige (10) aux vertèbres (12), dans lequel au moins l'un parmi une forme cible de la colonne (14) et une forme de la tige préformée (10) est définie dans des données de forme spécifiques au patient, une densité osseuse d'au moins l'une des vertèbres (12) est définie dans les données de densité osseuse spécifiques au patient, et dans lequel les données anatomiques spécifiques au patient sont fournies, le procédé étant effectué par un appareil (20) et consistant à:
calculer (1102), sur la base des données de forme et des données anatomiques, une distribution de force le long d'une longueur de la tige (10); et
générer (1104), sur la base des données de densité osseuse d'un vertèbre donnée (12) et de la distribution de force calculée, un résultat de planification pour le positionnement de fixation.

2. Procédé selon la revendication 1, dans lequel
le résultat de planification indique au moins l'une parmi une extension dans la vertèbre (12), une position d'attache de fixation le long d'une longueur de la tige (10) et au moins un paramètre de fixation d'une fixation (16) devant être insérée dans la vertèbre (12).

3. Procédé selon la revendication 1 ou 2, dans lequel
la génération (1104) du résultat de planification consiste à analyser une densité osseuse de la vertèbre (12) donnée, comme indiquée dans les données de densité osseuse, dans lequel le résultat de planification réponse au moins à un critère de stabilité prédéfinie au vu d'une force, indiquée par la distribution de force, agissant sur une fixation (16).

4. Procédé selon la revendication 3, dans lequel
l'au moins un critère de stabilité prédéfini est sélectionné parmi un ou plusieurs parmi un critère de stabilité osseuse, une exigence d'équilibre sagittal, un impact physiologique statique et un impact physiologique non statique.

5. Procédé selon l'une quelconque des revendications précédentes, dans lequel
le calcul sous-jacent aux données anatomiques spécifiques au patient de la distribution de force comprend un ou plusieurs paramètres anatomiques associés à la géométrique et un ou plusieurs paramètres anatomiques associés au poids.

6. Procédé selon l'une quelconque des revendications précédentes, consistant à
générer (1106) une instruction de navigation sur la base du résultat de planification.

7. Procédé selon au moins la revendication 2, consistant à
délivrer le résultat de planification qui comprend au moins un paramètre de fixation et une indication de la vertèbre donnée qui doit recevoir une fixation (16) ayant l'au moins un paramètre de fixation.

8. Procédé selon au moins l'une des revendication 2 et 7, dans lequel
l'au moins un paramètre de fixation indique au moins l'une parmi une longueur de fixation, un diamètre de fixation, un fil de fixation et un type de fixation.

9. Procédé selon l'une quelconque des revendications précédentes, dans lequel
au moins l'une des fixations (16) comprend une vis à os, une cheville à os et un fil K.

10. Procédé selon l'une quelconque des revendications précédentes, dans lequel
la distribution de force indique au moins l'un parmi un composant de vecteur de force qui s'étend de manière non coaxiale vers au moins l'une parmi l'extension de fixation dans la vertèbre (12) et une longueur d'une fixation (16) lorsqu'elle est attachée à la tige (10).

11. Procédé selon l'une quelconque des revendications précédentes, dans lequel
la distribution de force indique des vecteurs de force à différentes positions le long d'une longueur de la tige (10).

12. Procédé selon l'une quelconque des revendications précédentes, dans lequel
la distribution de force est en outre calculée sur la base de données génériques de tige.

13. Procédé selon la revendication 12, dans lequel
les données génériques de tige indiquent au moins l'une parmi une longueur de tige, un diamètre de tige et un paramètre de matériau de tige.

14. Procédé selon l'une quelconque des revendications précédentes, dans lequel
les données anatomiques indiquent une ou plusieurs parmi une inclinaison du bassin, une translation d'axe vertical sagittal, une asymétrie lordose lombaire/incidence pelvienne, une relation d'un fil à plomb C7 et un point central d'une inclinaison du bassin, un rapport taille/hauteur, un rapport taille/hanches, une répartition de masse le long d'un axe de la colonne vertébrale, un indice de masse corporelle, un poids corporel, une incidence pelvienne et une cyphose dorsale.

15. Produit programme d'ordinateur comprenant des parties de code de programme qui amènent un ordinateur à exécuter le procédé selon l'une quelconque des revendications précédentes lorsque le produit programme d'ordinateur est exécuté par le processeur.

16. Appareil (20) de planification du positionnement de fixations dans des vertèbres (12) qui doivent être stabilisées par une tige rachidienne (10), dans lequel au moins deux fixations (16) doivent accoupler la tige (10) aux vertèbres (12), dans lequel au moins l'une forme cible de la colonne (14) et une forme de la tige (10) est définie dans des données de forme spécifiques au patient, une densité osseuse d'au moins l'une des vertèbres (12) est définie dans les données de densité osseuse spécifiques au patient, et dans lequel les données anatomiques spécifiques au patient sont fournies, l'appareil étant configuré pour:
calculer (1102), sur la base des données de forme et des données anatomiques, une distribution de force le long d'une longueur de la tige (10); et
générer (1104), sur la base des données de densité osseuse d'un vertèbre (12) donnée et de la distribution de force calculée, un résultat de planification pour le positionnement de fixation.

17. Appareil (20) selon la revendication 16, configuré pour exécuter le procédé selon l'une quelconque des revendications 2 à 14.
